# EUROPEAN PATENT SPECIFICATION

(11) **EP 4 559 489 B1**
(45) Date of publication and mention of the grant of the patent: **28.10.2026**
(21) Application number: 24198293.3
(22) Date of filing: 06.12.2018
(51) Int. Cl.: A61M 1/00, A61M 3/00, A61M 35/00, A61F 13/00, A61F 13/02, A61F 13/0203, A61F 13/05

(54) **MANUALLY-OPERATED NEGATIVE PRESSURE WOUND THERAPY (NPWT) BANDAGE WITH IMPROVED PUMP EFFICIENCY, AUTOMATIC PRESSURE INDICATOR AND AUTOMATIC PRESSURE LIMITER**
HANDBETÄTIGTE UNTERDRUCKWUNDTHERAPIEBANDAGE MIT VERBESSERTER PUMPENEFFIZIENZ, AUTOMATISCHEM DRUCKANZEIGER UND AUTOMATISCHEM DRUCKBEGRENZER
BANDAGE DE THÉRAPIE DE PLAIE PAR PRESSION NÉGATIVE À COMMANDE MANUELLE (NPWT) À EFFICACITÉ DE POMPE AMÉLIORÉE, INDICATEUR DE PRESSION AUTOMATIQUE ET LIMITEUR DE PRESSION AUTOMATIQUE

(30) Priority: 06.12.2017 US 201762595398 P; 28.12.2017 US 201762611227 P
(43) Date of publication of application: 28.05.2025
(62) Document number(s) of the earlier application(s) in accordance with Art. 76 EPC: 18886192.6 / 3 720 518
(73) Proprietor: Cornell University, Ithaca, NY 14850 (US)
(72) Inventor: Johnson, Timothy, Freeport, 04032 (US); Sears, Charles, Boxford, 01921 (US); Albert, Sean, Barrington, 03825 (US); Tortoriello, David, Topsfield, 01983 (US); Soto, Orlando, Amesbury, 01913 (US)
(74) Representative: Christ, Niko

(56) References cited:
- WO-A1-2016/182977
- US-A- 5 549 584
- US-A1- 2015 202 354

## Description

### Field Of The Invention

This invention relates to bandages in general, and more particularly to negative pressure wound therapy (NPWT) bandages.

### Background Of The Invention

Bandages are used to provide wound care during healing. More particularly, bandages generally provide a covering for a wound so as to shield the wound from contaminants and microbes during healing. Most bandages also provide a closure feature to help hold the edges of the wound in close apposition during healing. Bandages also frequently include gauze or the like to receive exudates emerging from the wound during healing.

Negative pressure wound therapy (NPWT) bandages apply a negative pressure to a wound during healing. This negative pressure helps reduce the likelihood of contaminants and microbes entering the wound during healing, helps draw exudates from the wound during healing, and can promote beneficial biological responses at the wound site. More particularly, NPWT bandages typically comprise (i) an absorbent dressing configured to make a fully-sealed chamber around the perimeter of a wound ("the wound chamber"), (ii) a source of negative pressure, and (iii) a conduit extending between the fully-sealed wound chamber and the source of negative pressure. As a result of this construction, the absorbent dressing can be applied to a wound so as to create a fully-sealed chamber around the perimeter of the wound, and the source of negative pressure can apply a negative pressure to the fully- sealed wound chamber, such that any contaminants and microbes present at the wound site are drawn away from the wound, exudates are drawn out of the wound, and beneficial biological responses are promoted at the wound site.

Most NPWT bandages are part of a large, complex NPWT system, in the sense that (i) the absorbent dressings are generally fairly large (e.g., they are sized to cover large open wounds), (ii) the sources of negative pressure are generally fairly large, and formed and located separate from the absorbent dressings (e.g., the sources of negative pressure typically comprise electrically-powered suction pumps or vacuum cannisters), and (iii) the NPWT systems generally require substantial training to use. These NPWT systems also tend to be quite expensive.

Efforts have been made to provide a small, simplified and less expensive NPWT bandage where the source of negative pressure is integrated with the absorbent dressing. By way of example but not limitation, efforts have been made to provide an NPWT bandage where a manually-operated suction pump is integrated into the absorbent dressing.

Unfortunately, current NPWT bandages integrating a suction pump with the absorbent dressing tend to suffer from a variety of deficiencies, e.g., they have a complex design, and/or are expensive, and/or are complicated to use, and/or are bulky (including having a high profile), and/or cause additional trauma to the wound during use, and/or have poor pump efficiency, and/or lack a way of indicating the level of negative pressure created, and/or lack a way of limiting the level of negative pressure created, et c. In this latter respect it should be appreciated that where too high a level of negative pressure is created, the NPWT bandage can cause trauma to the patient, e.g., blistering, capillary leakage, etc.

Further prior art is known from US5549584, WO2016/182977 and US2015/202354.

Thus there is a need for a new and improved NPWT bandage which is simple, inexpensive, easy-to-use, small in size (including having a low profile), is atraumatic to the wound during use, has improved pump efficiency, incorporates an automatic pressure indicator for indicating the level of negative pressure created, and provides an automatic pressure limiter for limiting the level of negative pressure created .

### Summary Of The Invention

These and other objects of the invention are addressed by the provision and use of a new and improved NPWT bandage which is simple, inexpensive, easy-to-use, small in size (including having a low profile), is atraumatic to the wound during use, has improved pump efficiency, incorporates an automatic pressure indicator for indicating the level of negative pressure created, and provides an automatic pressure limiter for limiting the level of negative pressure created.

In the invention, there is provided a negative pressure wound therapy (NPWT) bandage for applying negative pressure to a wound as defined in claim 1.

Preferably, the NPWT bandage is configured so that when the pressure differential between the pressure of the fluid within said pump chamber and atmospheric pressure is below a predetermined threshold, said pump body of said pump assembly will assume a substantially fully expanded configuration, and when said pressure differential between the pressure of the fluid within said pump chamber and atmospheric pressure is above said predetermined threshold, said pump body of said pump assembly will assume a substantially fully collapsed configuration.

Even more preferably, the NPWT bandage is configured so that said pump body abruptly changes state between said substantially fully expanded configuration and said substantially fully collapsed configuration, and between said substantially fully collapsed configuration and said substantially fully expanded configuration, as said pressure differential crosses said predetermined threshold so as to effectively constitute a substantially "binary state" device .

### Brief Description Of The Drawings

These and other objects and features of the present invention will be more fully disclosed or rendered obvious by the following detailed description of the preferred embodiments of the invention, which is to be considered together with the accompanying drawings wherein like numbers refer to like parts, and further wherein:
Figs. 1-4 are schematic views showing a new and improved NPWT bandage, with Figs. 2 and 3 being exploded views ;

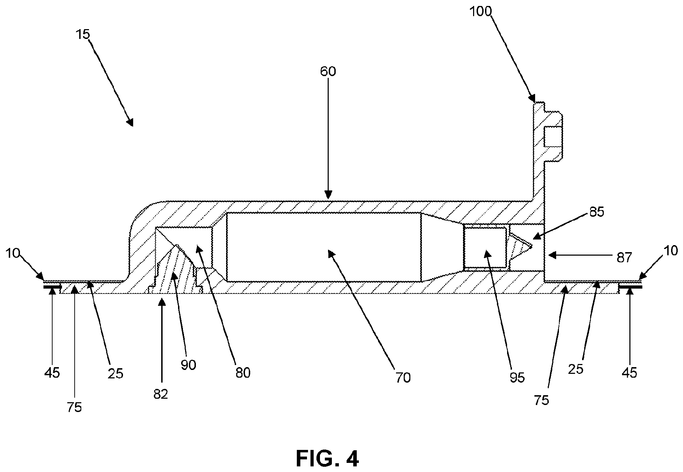

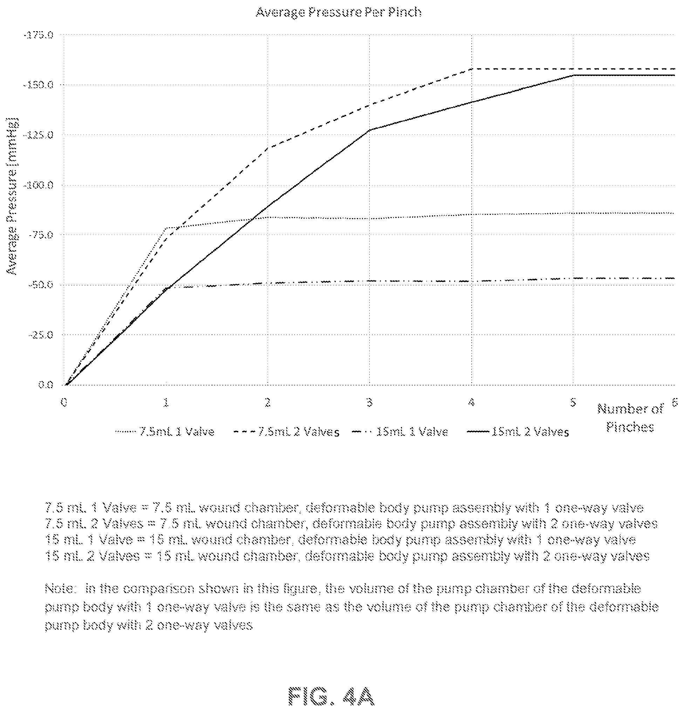

Fig. 4A is a schematic view showing, for two different size wound chambers (i.e., a 7.5 mL wound chamber and a 15 mL wound chamber), a maximum negative pressure that may be established with (i) a deformable pump body having two one-way valves (with one one-way valve being disposed on either side of the deformable pump body), and (ii) a deformable pump body having a single one-way valve (note: in the comparison shown in Fig. 4A, the volume of the pump chamber of the deformable pump body with 1 one-way valve is the same as the volume of the pump chamber of the deformable pump body with 2 one-way valves);
Figs. 5 and 6 are schematic views showing the pump body of the pump assembly of the NPWT bandage shown in Figs. 1-4 in its substantially fully expanded configuration (Fig. 5) and in its substantially fully collapsed configuration (Fig. 6);
Fig. 7 is a schematic view showing how the pump body of the pump assembly of the NPWT bandage shown in Figs. 1-4 abruptly changes state between its substantially fully expanded configuration and its substantially fully collapsed configuration;
Fig. 8 is a schematic view showing how the pump bodies of the pump assemblies of prior art NPWT bandages gradually change state between their substantially fully expanded configuration and their substantially fully collapsed configuration;
Figs. 9-16 are schematic views showing exemplary use of the new and improved NPWT bandage shown in Figs. 1-4 (note that in Figs. 11 and 14-16, removable cap 100 (see below) is removed from the figures for clarity of illustration) ;
Figs. 17-20 are schematic views showing another new and improved NPWT bandage; and
Figs. 21-25 are schematic views showing another new and improved NPWT bandage formed in accordance with the present invention.

Detailed Description Of The Preferred Embodiments The present invention comprises the provision and use of a new and improved NPWT bandage which is simple, inexpensive, easy-to-use, small in size (including having a low profile), is atraumatic to the wound during use, has improved pump efficiency, incorporates an automatic pressure indicator for indicating the level of negative pressure created, and provides an automatic pressure limiter for limiting the level of negative pressure created.

### The Manually-Operated Negative Pressure Wound Therapy

### (NPWT) Bandage In General

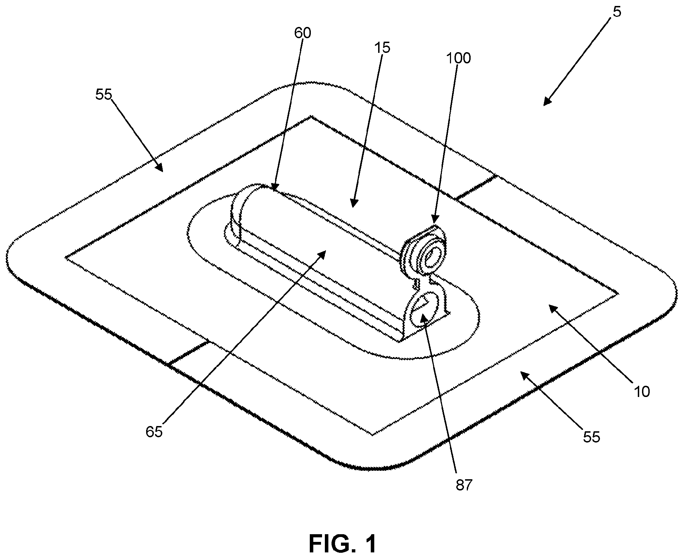

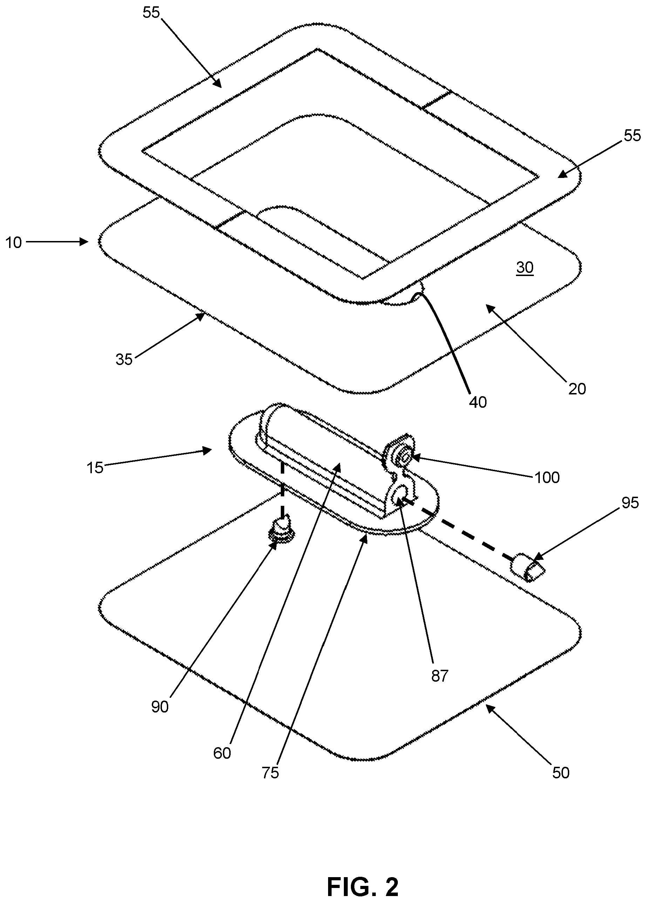

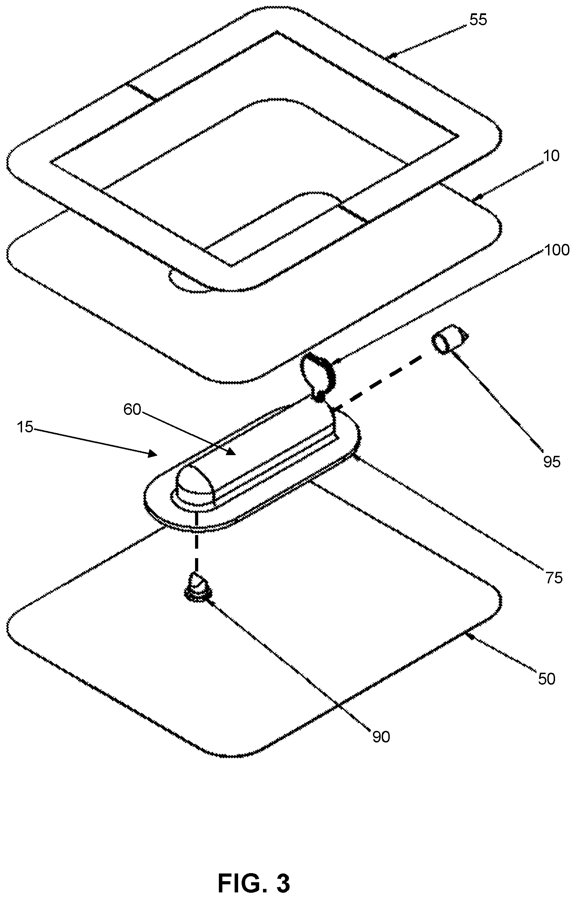

More particularly, and looking first at Figs. 1- 4, there is shown a manually-operated negative pressure wound therapy (NPWT) bandage 5 having improved pump efficiency, an automatic pressure indicator for indicating the level of negative pressure created, and an automatic pressure limiter for limiting the level of negative pressure created.

NPWT bandage 5 generally comprises a membrane (or sheet) 10 and a pump assembly 15.

As will hereinafter be discussed, membrane 10 is configured to make a fully-sealed chamber around the perimeter of a wound, whereby to define a wound chamber .

And as will hereinafter be discussed, pump assembly 15 is configured to apply a negative pressure to the fully-sealed wound chamber, such that any contaminants and microbes present at the wound site are drawn away from the wound, exudates are drawn out of the wound, and beneficial biological responses are promoted at the wound site. Significantly, pump assembly 15 is designed to provide improved pump efficiency, an automatic pressure indicator for indicating the level of negative pressure created, and an automatic pressure limiter for limiting the level of negative pressure created, as will hereinafter be discussed .

### The Membrane

More particularly, membrane 10 comprises a flat planar sheet 20 formed out of a flexible, substantially air-impermeable material, e.g., Tegaderm from 3M Company (which has also been known as the Minnesota Mining and Manufacturing Company), so that it can conform to body contours and form a substantially air-tight chamber around the perimeter of a wound (i.e., the wound chamber) . Membrane 10 is characterized by a wound-side surface 25 and an atmosphere-side surface 30. Membrane 10 is also characterized by an outer perimeter 35 and an inner opening 40.

An adhesive 45 is preferably disposed on wound- side surface 25 of membrane 10. A release liner 50 is preferably disposed on wound-side surface 25 atop adhesive 45 so as to keep adhesive 45 covered until use .

A removable stiffener 55 is preferably disposed on atmosphere-side surface 30 of membrane 10.

Removable stiffener 55 serves to facilitate manipulation of NPWT bandage 5 (and particularly membrane 10) during removal of the NPWT bandage from its sterile packaging and during positioning of the NPWT bandage about a wound. Removable stiffener 55 is intended to be removed from membrane 10 once NPWT bandage 5 has been secured about the wound site.

Removable stiffener 55 may be provided as a single element or, more preferably, removable stiffener 55 is provided as a pair of elements so as to facilitate removal from membrane 10 after NPWT bandage 5 has been secured about the wound site.

### The Pump Assembly

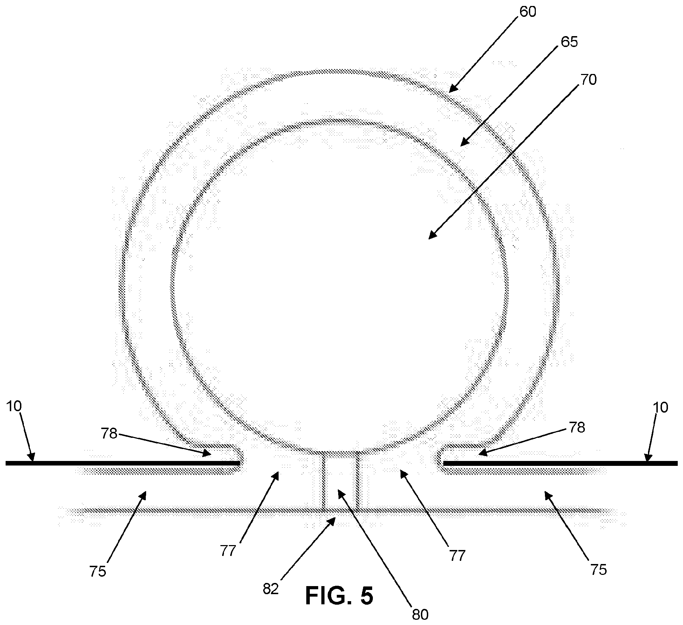

Pump assembly 15 comprises a pump body 60 having a generally cylindrical shape and comprising a side wall 65 and an inner chamber 70. Pump body 60 is formed out of a resilient material, e.g., silicone, such that side wall 65 may be compressed inwardly by the application of an external force (e.g., squeezing by the thumb and forefinger of a user) and will then attempt to return to its original uncompressed state when the external force is removed. A pump flange 75 is preferably formed on one side of pump body 60. As will hereinafter be discussed in further detail, pump body 60 extends through inner opening 40 of membrane 10, and the upper surface of pump flange 75 is secured to the wound-side surface 25 of membrane 10 so that pump assembly 15 is secured to, and carried by, membrane 10. Pump flange 75 is preferably formed out of a flexible material so that it can conform (to at least a limited extent) to body contours. In one form of the invention, pump body 60 and pump flange 75 are formed integral with one another out of the same material, e.g., silicone. In one preferred form of the invention, side wall 65 of pump body 60 and pump flange 75 merge at a neck 77 (Fig. 5) . And in one preferred form of the invention, neck 77 has a relatively small width relative to the full diameter of pump body 60, with recesses 78 extending inwardly between membrane 10 and pump body 60, such that pump body 60 is mounted to pump flange 75 but is still free to radially compress/radially expand with minimal interference from pump flange 75. A wound-side passageway 80 is formed in pump body 60 and communicates with inner chamber 70. Wound-side passageway 80 opens on the exterior of pump body 60 at a wound-side port 82. An atmosphere-side passageway 85 is formed in pump body 60 and also communicates with inner chamber 70. Atmosphere-side passageway 85 opens on the exterior of pump body 60 at an atmosphere-side port 87.

A wound-side one-way valve 90 is disposed in wound-side passageway 80 and is configured to permit fluid to enter inner chamber 70 through wound-side passageway 80 but to prevent fluid from exiting inner chamber 70 through wound-side passageway 80.

An atmosphere-side one-way valve 95 is disposed in atmosphere-side passageway 85 and is configured to permit fluid to exit inner chamber 70 through atmosphere-side passageway 85 but to prevent fluid from entering inner chamber 70 through atmosphere-side passageway 85.

As a result of this construction, when pump body 60 of pump assembly 15 is manually squeezed (e.g., by applying a compressive force to side wall 65 of pump body 60 with the thumb and forefinger of a user), fluid (e.g., air, liquid, etc.) within inner chamber 70 will be forced out of inner chamber 70 via atmosphere-side passageway 85, and when pump body 60 of pump assembly 15 is thereafter released (e.g., by relaxing the compressive force applied to side wall 65 of pump body 60 by the thumb and forefinger of a user), fluid (e.g., air, liquid, etc.) below wound- side surface 25 of membrane 10 (e.g., air, liquid, etc. within the wound chamber) will be drawn into inner chamber 70 through wound-side passageway 85 as the resilient side wall of the pump body returns to its uncompressed state.

Note that when pump body 60 of pump assembly 15 is manually squeezed, fluid (e.g., air, liquid, etc.) within inner chamber 70 is prevented from exiting inner chamber 70 through wound-side passageway 80 due to the one-way operation of wound-side one-way valve 90, and when pump body 60 of pump assembly 15 is thereafter released, air from the atmosphere is prevented from being drawn into inner chamber 70 through atmosphere-side passageway 85 due to the one way operation of atmosphere-side one-way valve 95.

Thus it will be appreciated that repeatedly manually squeezing and releasing pump body 60 of pump assembly 15 will apply suction to the wound chamber disposed below wound-side surface 25 of membrane 10, whereby to create negative pressure at the wound site.

It should be appreciated that the present invention's approach of providing a pump assembly utilizing two one-way valves disposed on either side of a deformable pump body with an in-line configuration (i.e., wound-side one-way valve 90 and atmosphere-side one-way valve 95 disposed on either side of deformable pump body 60) provides a number of significant advantages which are not achievable with the prior art's approach of providing a deformable pump body utilizing a single one-way valve.

More particularly, and as will hereinafter be discussed, the present invention's approach of providing a pump assembly utilizing two one-way valves disposed on either side of a deformable pump body with an in-line configuration (i.e., wound-side one-way valve 90 and atmosphere-side one-way valve 95 disposed on either side of deformable pump body 60) allows substantially the same maximum negative pressure to be established at the wound site regardless of the size of the wound chamber. This is not achievable with the prior art's approach of providing a deformable pump body utilizing a single one-way valve.

In addition, the present invention's approach of providing a pump assembly utilizing two one-way valves disposed on either side of a deformable pump body with an in-line configuration (i.e., wound-side one-way valve 90 and atmosphere-side one-way valve 95 disposed on either side of deformable pump body 60) allows a greater constant selected maximum negative pressure to be achieved at the wound site than can be achieved at the wound site using a deformable pump body with a single one-way valve (which is reflective of the prior art's approach) .

More particularly, Fig. 4A, shows, for two different size wound chambers (i.e., a 7.5 mL wound chamber and a 15 mL wound chamber), a maximum negative pressure that may be established with (i) a deformable pump body having two one-way valves (with one one-way valve being disposed on either side of the deformable pump body), and (ii) a deformable pump body having a single one-way valve (note: in the comparison shown in Fig. 4A, the volume of the pump chamber of the deformable pump body with 1 one-way valve is the same as the volume of the pump chamber of the deformable pump body with 2 one-way valves) .

Inherent in Fig. 4A are a number of significant aspects of the present invention. First, Fig. 4A shows that using a deformable pump body having two one-way valves (with one one-way valve being disposed on either side of the deformable pump body) to evacuate the wound chamber lets you establish substantially the same maximum negative pressure in the wound chamber regardless of the size of the wound chamber (i.e., it yields approximately -150.0 mm Hg for a 7.5 mL wound chamber and approximately - 150.0 mm Hg for a 15 mL wound chamber), whereas using a deformable pump body having a single one-way valve does not (i.e., it yields approximately -80.0 mm Hg for a 7.5 mL wound chamber and approximately -50.0 mm Hg for a 15 mL wound chamber) . Thus, the NPWT bandage of the present invention allows substantially the same maximum negative pressure to be established at the wound site regardless of the size of the wound chamber, whereas prior art NPWT bandages do not.

This unique feature of the present invention is clinically significant, inasmuch as (i) it is generally desirable to establish a selected maximum negative pressure at the wound site (e.g., between about 60 mm Hg and about 180 mm Hg) , and (ii) it is generally difficult to know in advance the volume of the wound chamber (e.g., due to variations in medical applications, variations in patient anatomy, etc.) . Thus, inasmuch as the NPWT bandage of the present invention allows substantially the same maximum nega tive pressure to be established at the wound site regardless of the size of the wound chamber, the present invention allows the NPWT bandage to be engineered in advance (e.g., at the time of manufacture) to establish a selected maximum negative pressure at the wound site, whereas prior art NPWT bandages do not.

Second, Fig. 4A shows that using a deformable pump body having two one-way valves (with one one-way valve being disposed on either side of the deformable pump body) to evacuate the wound chamber lets you establish a substantially higher maximum negative pressure in the wound chamber (i.e., it yields approximately -150.0 mm Hg for a 7.5 mL wound chamber and approximately -150.0 mm Hg for a 15 mL wound chamber) than can be established using a deformable pump body having a single one-way valve (i.e., it yields approximately -80.0 mm Hg for a 7.5 mL wound chamber and approximately -50.0 mm Hg for a 15 mL wound chamber) . Thus, the NPWT bandage of the present invention allows a substantially higher maximum negative pressure to be established at the wound site.

Note also that the pressure within inner chamber 70 of pump body 60 is generally equal to the pressure below wound-side surface 25 of membrane 10 (i.e., the pressure within inner chamber 70 of pump body 60 is generally equal to the pressure within the wound chamber) . In one preferred form of the invention, pump assembly 15 also comprises a removable cap 100.

Removable cap 100 is configured to selectively close off atmosphere-side passageway 85 to fluid flow when removable cap 100 is inserted into atmosphere-side passageway 85 so as to close off atmosphere-side port 87.

Pump assembly 15 is mounted to membrane 10 such that pump assembly 15 is carried by membrane 10. More particularly, pump assembly 15 is mounted to membrane 10 by (i) passing pump body 60 of pump assembly 15 through inner opening 40 of membrane 10, (ii) bringing pump flange 75 up against wound-side surface 25 of membrane 10, and then (iii) adhering pump flange 75 to wound-side surface 25 of membrane 10 (e.g., by bonding, gluing, etc.) . Note that pump assembly 15 and membrane 10 make a substantially air-tight connection .

Significantly, pump body 60 of pump assembly 15 is carefully configured to provide (i) improved pump efficiency, (ii) an automatic pressure indicator for indicating the level of negative pressure created, and (iii) an automatic pressure limiter for limiting the level of negative pressure created, as will hereinafter be discussed.

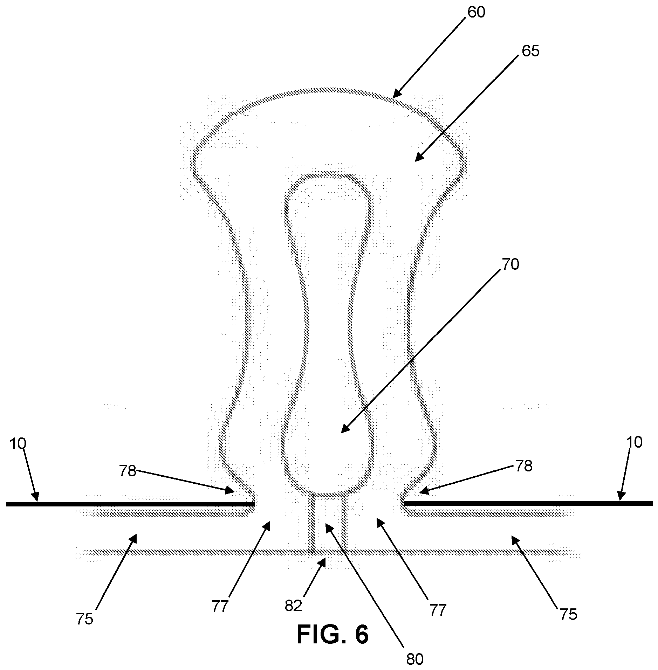

More particularly, pump body 60 of pump assembly 15 is specifically configured so that the pump body will abruptly change state between (i) a substantially fully expanded configuration where side wall 65 of pump body 60 and inner chamber 70 of pump body 60 have a substantially circular cross-section (see Fig. 5) when the pressure differential between the pressure of the fluid within inner chamber 70 and atmospheric pressure is below a given threshold, and (ii) a substantially fully collapsed configuration w here side wall 65 of pump body 60 bows inwardly (see Fig. 6) when the pressure differential between the pressure of the fluid within inner chamber 70 and atmospheric pressure exceeds a given threshold.

Specifically, when the pressure differential between the pressure of the fluid within inner chamber 70 and atmospheric pressure is below a given threshold, pump body 60 of pump assembly 15 will assume its substantially fully expanded configuration (Fig. 5), and when the pressure differential between the pressure of the fluid within inner chamber 70 and atmospheric pressure is above a given threshold, pump body 60 of pump assembly 15 will assume its substantially fully collapsed configuration (Fig. 6) .

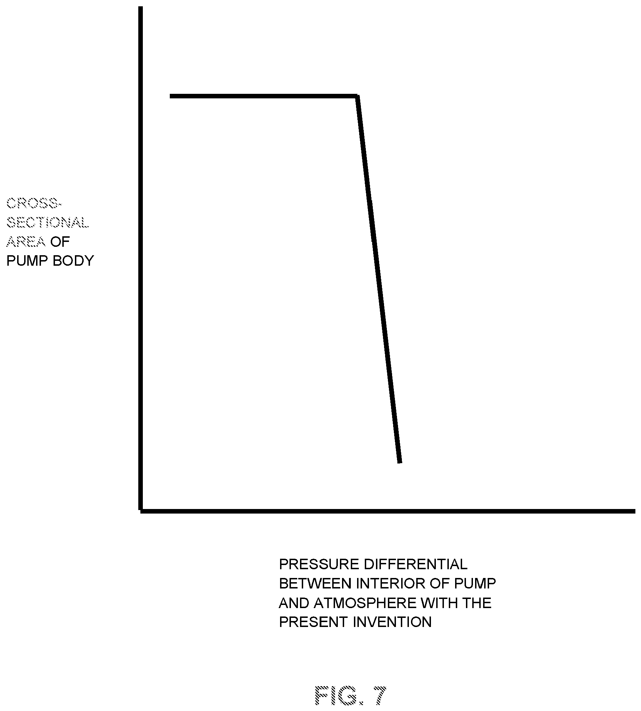

Significantly, pump body 60 of pump assembly 15 is configured so that it will abruptly change state between its substantially fully expanded configuration (Fig. 5) and its substantially fully collapsed configuration (Fig. 6) when the pressure differential between the pressure of the fluid within inner chamber 70 and atmospheric pressure crosses the aforementioned given threshold. See Fig. 7, which is a graph showing the relationship between the diameter of side wall 65 of pump body 60 and the pressure differential between the pressure of a fluid within inner chamber 70 and atmospheric pressure. Thus, pump assembly 15 is specifically configured to essentially behave as a substantially "binary state" device - it is either substantially fully expanded (Fig. 5) or substantially fully collapsed (Fig. 6) . In this respect it should be appreciated that, as used herein, the term "substantially "binary state" device" is intended to refer to a device which is inclined to assume either a substantially fully expanded condition or a substantially fully collapsed condition and, as used herein, the term "substantially "binary state" behavior" is intended to refer to the inclination of a device to assume either a substantially fully expanded condition or a substantially fully collapsed condition.

Note that the substantially "binary state" behavior of pump body 60 is a consequence of forming the pump body with a side wall 65 having a substantially circular cross-section, which gives the pump body an "over-the-center" deformation characteristic, i.e., the side wall of pump body 60 has a "failure" mode where it abruptly transitions from its substantially fully expanded configuration to its substantially fully collapsed configuration, and has a "restoration" mode where it abruptly transitions from its s ubstantially fully collapsed configuration to its substantially fully expanded configuration.

See Fig. 7. Note that by forming pump assembly 15 so that side wall 65 of pump body 60 and pump flange 75 merge at a neck 77 (Fig. 5), with neck 77 having a relatively small width relative to the full diameter of pump body 60, and with recesses 78 extending inwardly between membrane 10 and pump body 60, pump body 60 has a substantially circular cross-section over substantially its entire circumference, with pump body 60 free to radially compress/radially expand with minimal interference from pump flange 75, so that pump body 60 can exhibit substantially "binary state" behavior.

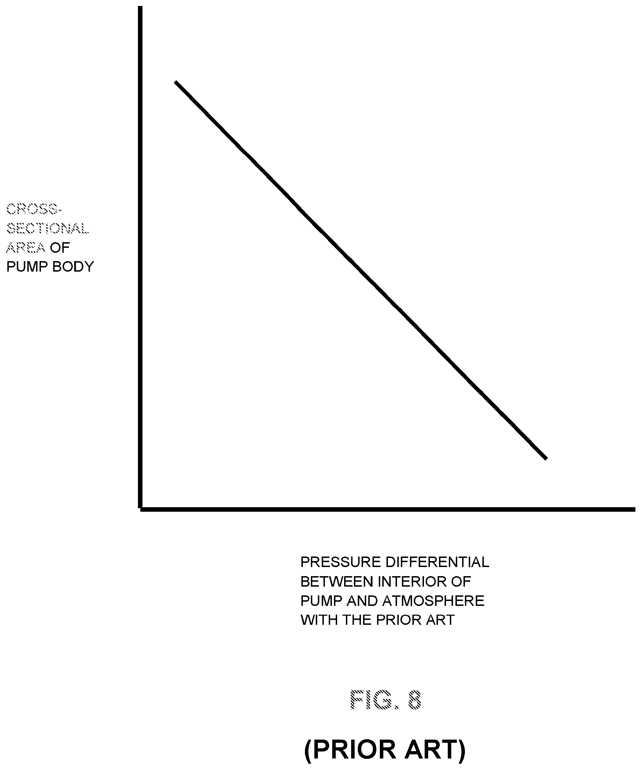

Note also that the prior art approaches of forming the pump body with dome-like or square pump configurations does not provide the pump body with an abrupt change of state - rather, these prior art dome like or square pump configurations provide the pump body with a more gradual change of state between an expanded configuration and a collapsed configuration when the pressure differential between the pressure of the fluid within an inner chamber and atmospheric pressure changes. See Fig. 8, which is a graph showing the relationship between the diameter of the side wall of a pump body having a dome-like or square configuration and the pressure differential between the pressure of a fluid within an inner chamber of the pump body and atmospheric pressure.

As a result of deliberately configuring side wall 65 of pump body 60 of pump assembly 15 to exhibit this abrupt change of state, pump assembly 15 is able to provide improved pump efficiency, an automatic pressure indicator for indicating the level of negative pressure created, and an automatic pressure limiter for limiting the level of negative pressure created .

More particularly, by configuring pump body 60 of pump assembly 15 so that it will abruptly change state between its substantially fully expanded configuration and its substantially fully collapsed configuration when the pressure differential between the pressure of a fluid within inner chamber 70 and atmospheric pressure crosses a given threshold, pump assembly 15 effectively returns to its substantially fully expanded configuration as long as the pressure differential between the pressure of the fluid within inner chamber 70 and atmospheric pressure is below the given threshold. As a result, so long as the pressure differential between the fluid within inner chamber 70 and atmospheric pressure is below the given threshold, pump assembly 15 returns to its substantially fully expanded configuration between compressions (i.e., squeezes), and hence remains fully efficient as it applies a negative pressure to the wound chamber. This is in contrast to the performance of prior art devices where the pump body exhibits a gradual change of state between an expanded configuration and a collapsed configuration when the pressure differential between the pressure of a fluid within the inner chamber of the pump assembly changes, which makes the pump assembly progressively less efficient as it reduces the pressure within the wound chamber. This is because the pump body will progressively return less and less to its fully expanded configuration as negative pressure is created in the wound chamber, so that the pump assembly is able to evacuate less and less fluid with each squeeze of the pump body. In other words, with prior art devices, the pump assembly becomes less and less efficient as negative pressure is created in the wound chamber.

In a related manner, by configuring pump body 60 so that it will abruptly change state between its substantially fully expanded configuration and its substantially fully collapsed configuration when the pressure differential between the pressure of the fluid within inner chamber 70 and atmospheric pressure crosses a given threshold, pump assembly 15 is able to function as an automatic pressure indicator for indicating the level of negative pressure created, i.e., so long as pump body 60 of pump assembly 15 returns to its substantially fully expanded configuration between squeezes, it will be readily apparent to an observer that the pressure within inner chamber 70 (and hence the pressure within the wound chamber) will be less than a given level. This is in marked contrast to the performance of prior art devices where pump body 60 provides a gradual change of state between an expanded configuration and a collapsed configuration when the pressure differential between the pressure of a fluid within an inner chamber of the pump assembly changes, in which case the pump assembly is not able to function as an automatic pressure indicator for indicating the level of negative pressure created.

And also in a related manner, by configuring pump body 60 so that it will abruptly change state between its substantially fully expanded configuration and its substantially fully collapsed configuration when the pressure differential between the pressure of a fluid within inner chamber 70 and atmospheric pressure crosses a given threshold, pump assembly 15 is able to function as an automatic pressure limiter for limiting the level of negative pressure created since, as soon as pump body 60 assumes its substantially fully collapsed configuration, pump assembly 15 is no longer able to pump fluid from the wound chamber, essentially deactivating the pump assembly. This is in marked contrast to the performance of prior art devices where the pump body provides a gradual change of state between an expanded configuration and a collapsed configuration when the pressure differential between the pressure of a fluid within an inner chamber changes, since the pump assembly is not effectively deactivated at a given pressure differential.

It should be appreciated that the pressure differential required to transition pump body 60 between its substantially fully-expanded configuration and its substantially fully-collapsed configuration (i.e., the aforementioned "given threshold") may be "tuned" (i.e., tailored) to a particular level by varying one or more characteristics of pump body 60, e.g., by forming side wall 65 of pump body 60 out of a material having a particular durometer, by adjusting the thickness of side wall 65 of pump body 60, by adjusting the diameter of inner chamber 70 of pump body 60, etc.

In general, it has been found that excellent therapeutic results may be achieved when the pressure differential required to transition pump body 60 between its substantially fully-expanded configuration and its substantially fully-collapsed configuration (i.e., the aforementioned "given threshold") is between about 60 mm Hg and about 180 mm Hg. In other words, it has been found that excellent therapeutic results may be achieved where pump body 60 transitions between its substantially fully-expanded configuration (Fig. 5) and its substantially fully-collapsed configuration (Fig. 6) at a negative pressure of between about 60 mm Hg and about 180 mm Hg. It is believed that where pump body 60 transitions between its two states at a lower pressure (i.e., where pump body 60 transitions at a negative pressure lower than about 60 mm Hg) , not enough suction is provided at the wound site to effectively draw contaminants and microbes away from the wound site and/or to effectively draw exudates away from the wound site a nd/or to promote beneficial biological responses at the wound site. It is also believed that where pump body 60 transitions between its two states at a higher pressure (i.e., where pump body 60 transitions at a negative pressure higher than about 180 mm Hg) , the suction provided at the wound site may cause trauma to the tissue (e.g., blistering, capillary leakage, etc.).

In one preferred form of the invention, pump body 60 of pump assembly 15 is configured so that it abruptly transitions between its substantially fully expanded configuration and its substantially fully collapsed configuration when the pressure differential between the pressure of the fluid within inner chamber 70 and atmospheric pressure exceeds 80 mm Hg. Thus, in this form of the invention, as long as the negative pressure within the wound chamber is less than 680 mm Hg (assuming atmospheric pressure is 760 mm Hg) , pump assembly 15 returns to its substantially fully expanded configuration between squeezes of the pump body and maintains its pump efficiency as it applies suction to the wound chamber, and as soon as the negative pressure within the wound chamber exceeds 680 mm Hg (assuming atmospheric pressure is 760 mm Hg) , pump assembly 15 will assume its substantially fully collapsed configuration, acting as an automatic pressure indicator to indicate that the level of negative pressure created at the wound site has exceeded 80 mm Hg and automatically deactivating pump assembly 15 so that the level of negative pressure created at the wound site cannot exceed 80 mm Hg.

Note that inasmuch as pump body 60 of pump assembly 15 has a substantially cylindrical configuration, NPWT bandage 5 has a low profile.

Note also that inasmuch as pump body 60 of pump assembly 15 is configured to be squeezed between the thumb and forefinger of a user, the compressive force being applied to pump body 60 is applied parallel to the surface of the skin, so that no trauma is applied to the wound during use (i.e., during pumping of pump assembly 15) . This is in marked contrast to prior art NPWT bandages which employ a dome-like configuration and require the compressive force to be applied toward the wound.

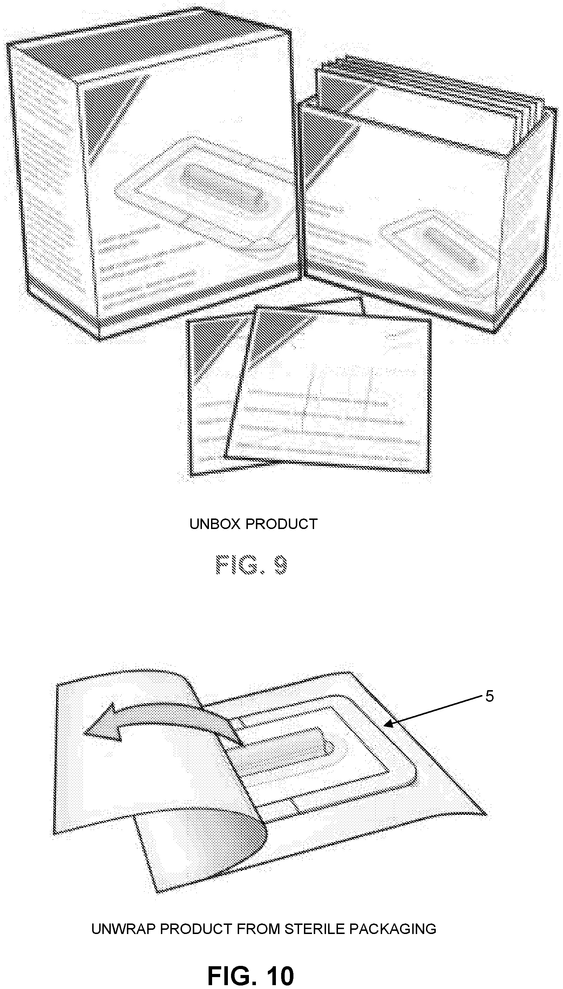

Exemplary Use In one preferred form of the invention, and looking now at Figs. 9-16, NPWT bandage 5 is intended to be used as follows.

First, an NPWT bandage 5 is removed from its box. In one form of the invention, each individual NPWT bandage 5 is contained in a separate sterile package, with multiple sterile packages contained in a box.

See Fig. 9.

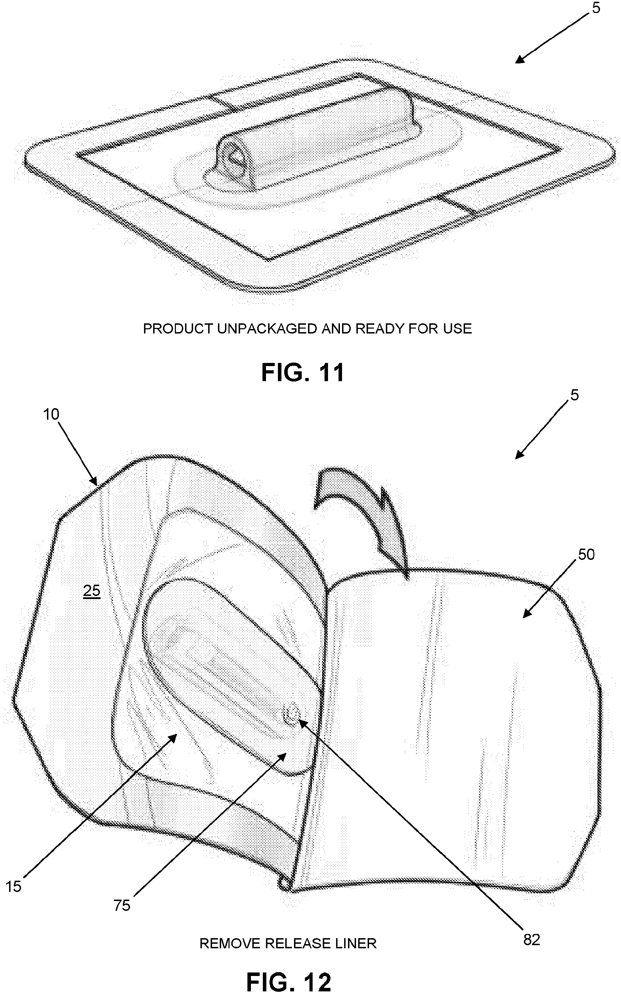

Next, an NPWT bandage 5 is removed from its sterile package (Fig. 10) so as to be ready for use (Fig. 11).

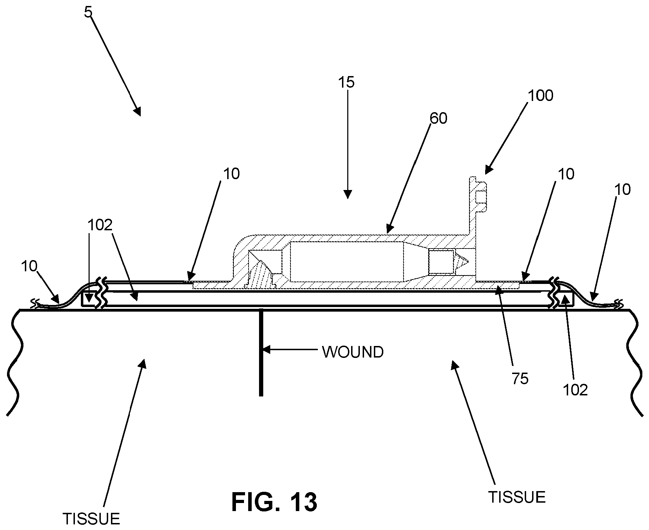

In order to apply NPWT bandage 5 to the wound site, release liner 50 is removed from wound-side surface 25 of membrane 10. See Fig. 12. Then NPWT bandage 5 is positioned against the skin of a patient so that wound-side surface 25 of membrane 10 is positioned against the wound, with adhesive 45 securing NPWT bandage 5 to the skin of the patient, thereby forming a substantially air-tight seal with the skin of the patient about the perimeter of the wound chamber. See Fig. 13.

Note that when NPWT bandage 5 is applied to the skin of the patient, wound-side port 82 of wound-side passageway 80 of pump assembly 15 is open to the wound chamber .

Note also that a layer of gauze (or other absorbent wound dressing) 102 may be placed on the wound site prior to placing NPWT bandage 5 on the skin of the patient, so that the layer of gauze (or other absorbent wound dressing) is interposed between the wound and wound-side passageway 80 of pump assembly 15. As a result, exudate emerging from the wound will be taken up by the gauze (or other absorbent wound dressing) . Note that, if desired, the layer of gauze (or other absorbent wound dressing) 102 may be mounted to (i.e., secured to) the wound-side surface of membrane 10, e.g., such as at the time of manufacture, so that the layer of gauze (or other absorbent wound dressing) 102 is carried to the wound site by NPWT bandage 5 and is applied to the wound at the same time as the NPWT bandage 5.

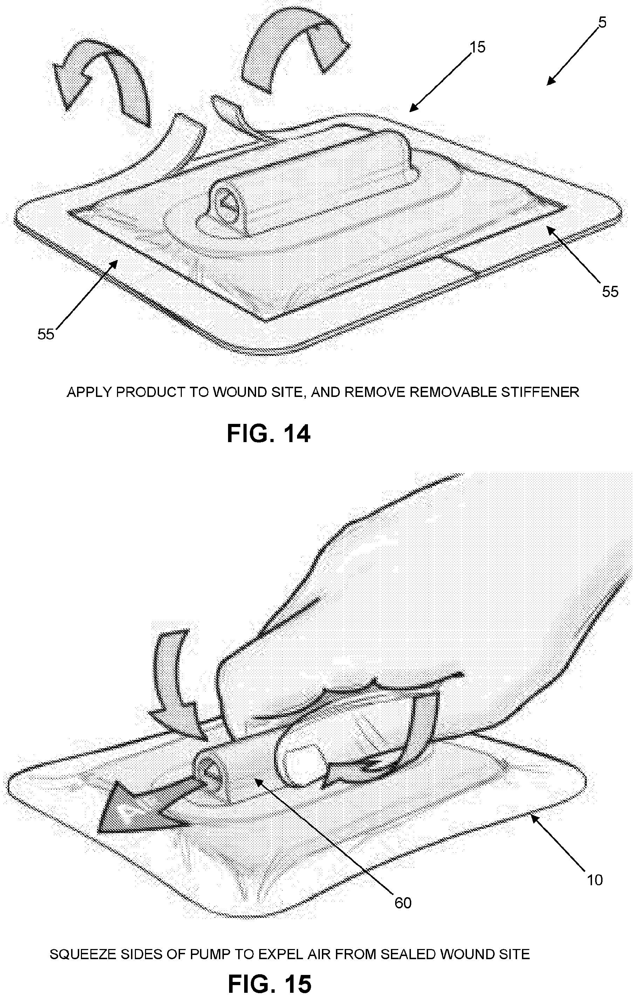

Next, with NPWT bandage 5 secured to the skin of the patient, removable stiffener 55 is removed from atmosphere-side surface 30 of membrane 10. See Fig. 14.

At this point, NPWT bandage 5 may be used to apply negative pressure to the wound chamber. This is done by squeezing side wall 65 of pump body 60 between the thumb and forefinger of a user so as to compress pump body 60 into its substantially fully collapsed configuration, whereby to expel fluid (e.g., air, liquid, etc.) from inner chamber 70 of pump body 60 via atmosphere-side passageway 85 and atmosphere-side one-way valve 95. See Fig. 15. Note that fluid in inner chamber 70 of pump body 60 is prevented from exiting inner chamber 70 through wound-side passageway 80 due to the presence of wound-side one-way valve 90. Then side wall 65 of pump body 60 is released, allowing the resilient pump body 60 to return to its substantially fully expanded configuration, thereby creating a negative pressure within inner chamber 70 and wound-side passageway 80, such that fluid below wound-side surface 25 of membrane 10 (e.g., fluid within the wound chamber) is drawn into inner chamber 70 through wound-side passageway 85 and wound-side one-way valve 90. Note that air in the atmosphere is prevented from entering inner chamber 70 through atmosphere-side passageway 85 due to the presence of atmosphere-side one-way valve 95.

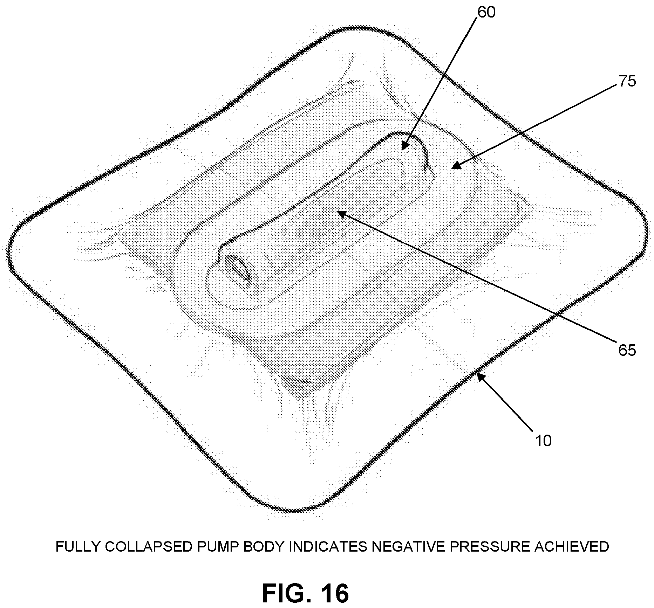

This process of squeezing and releasing side wall 65 of pump body 60 is repeated until pump body 60 of pump assembly 15 remains in its substantially fully collapsed configuration (i.e., side wall 65 of pump body 60 bows inwardly) even when side wall 65 of pump body 60 is not being manually compressed. See Fig. 16. When pump body 60 of pump assembly 15 remains in its substantially fully collapsed configuration even when side wall 65 of pump body 60 is not being manually compressed, an observer will know that the pressure differential between the pressure of the fluid within inner chamber 70 (and the wound chamber) and atmospheric pressure exceeds the desired threshold, indicating that the desired level of negative pressure has been achieved at the wound site. Note that when pump body 60 of pump assembly 15 remains in its substantially fully collapsed configuration even when side wall 65 of pump body 60 is not being manually compressed, pump assembly 15 will have been effectively deactivated, since it will be impossible to continue using the pump assembly with side wall 65 in its substantially fully collapsed configuration .

At this point removable cap 100 may be used to seal atmosphere-side port 87 of atmosphere-side passageway 85.

NPWT bandage 5 is left in place on the wound for an appropriate period of time (e.g., a few days) so as to shield the wound from contaminants and microbes during healing, draw exudates out of the wound, and promote beneficial biological responses at the wound site. In the event that leakage should cause the negative pressure created in the wound chamber to fall below the given threshold (which will be apparent to an observer by virtue of the fact that side wall 65 of pump body 60 will return to its substant ially fully- expanded configuration), atmosphere-side port 87 of atmosphere-passageway 85 may be unsealed (i.e., by removing removable cap 100) and then pump assembly 15 may be used in the manner discussed above to re establish the desired negative pressure in the wound chamber (i.e., by repeatedly squeezing and releasing side wall 65 of pump body 60) . When appropriate, NPWT bandage 5 may be removed from the skin of the patient by simply peeling membrane 10 away from the skin of the patient.

Pump Body With Notches To Enhance The Substantially "Binary State" Behavior Of The Pump Body As noted above, pump body 60 of pump assembly 15 is preferably specifically configured so that the pump body will abruptly change state between (i) a substantially fully expanded configuration where side wall 65 of pump body 60 and inner chamber 70 of pump body 60 have a substantially circular cross-section (see Fig. 5) when the pressure differential between the pressure of the fluid within inner chamber 70 and atmospheric pressure is below a given threshold, and (ii) a substantially fully collapsed configuration where side wall 65 of pump body 60 bows inwardly (see Fig. 6) when the pressure differential between the pressure of the fluid within inner chamber 70 and atmospheric pressure exceeds a given threshold.

As also noted above, this substantially "binary state" behavior of pump body 60 is achieved by forming the pump body with a substantially circular cross- section, which gives the body an "over-the-center" deformation characteristic, i.e., so that the side wall of pump body 60 has a "failure" mode where it abruptly transitions from a substantially fully expanded configuration to a substantially fully collapsed configuration, and has a "restoration" mode where it abruptly transitions from a substantially fully collapsed configuration to a substantially fully expanded configuration. See Fig. 7. As noted above, by forming pump assembly 15 so that side wall 65 of pump body 60 and pump flange 75 merge at a neck 77 (Fig. 5), with neck 77 having a relatively small width relative to the full diameter of pump body 60, and with recesses 78 extending inwardly between membrane 10 and pump body 60, pump body 60 has a substantially circular cross-section over substantially its entire circumference, with pump body 60 free to radially compress/radially expand with minimal interference from pump flange 75, so that pump body 60 can exhibit substantially "binary state" behavior.

If desired, pump body 60 can be modified so as to enhance the substantially "binary state" behavior of the pump body.

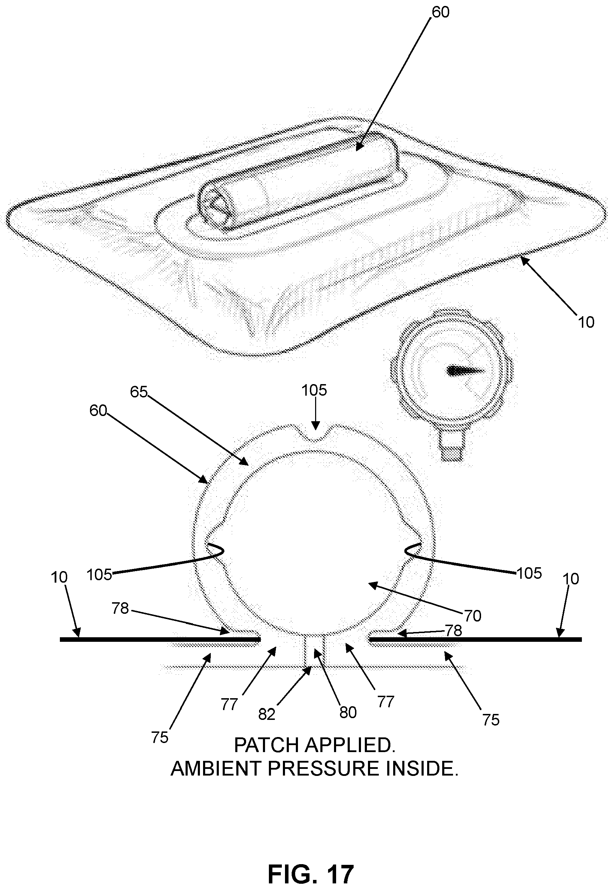

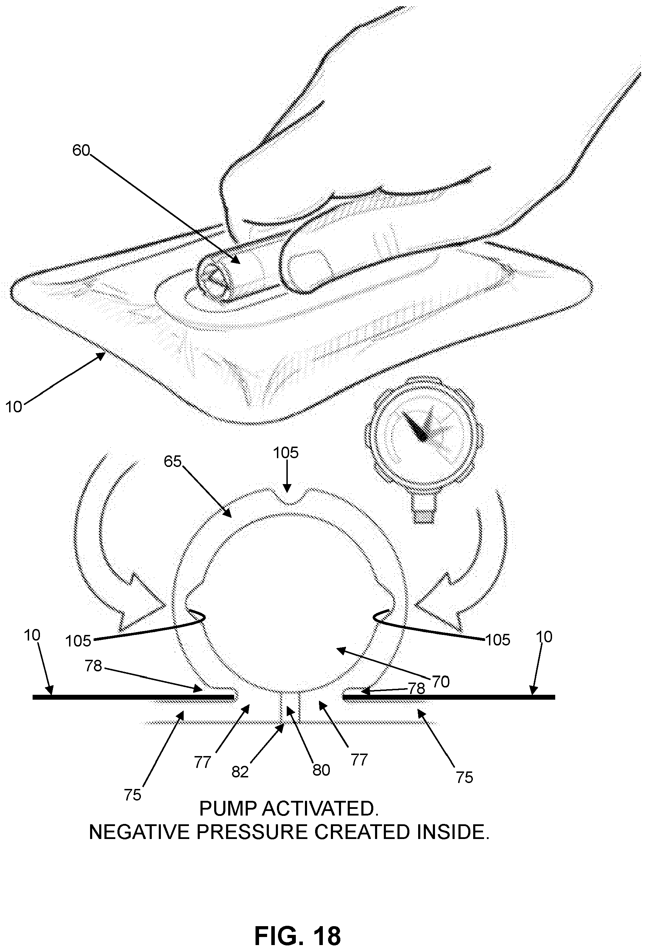

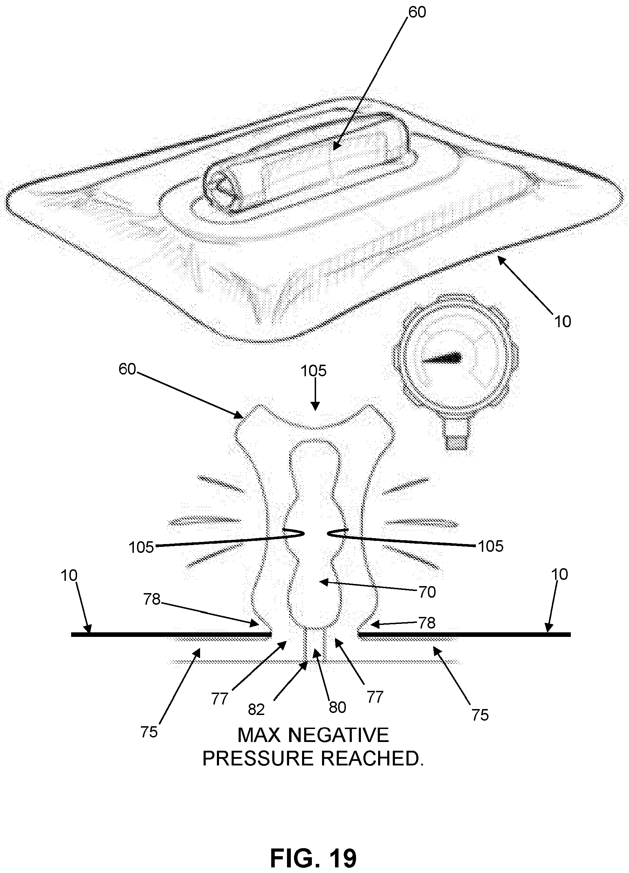

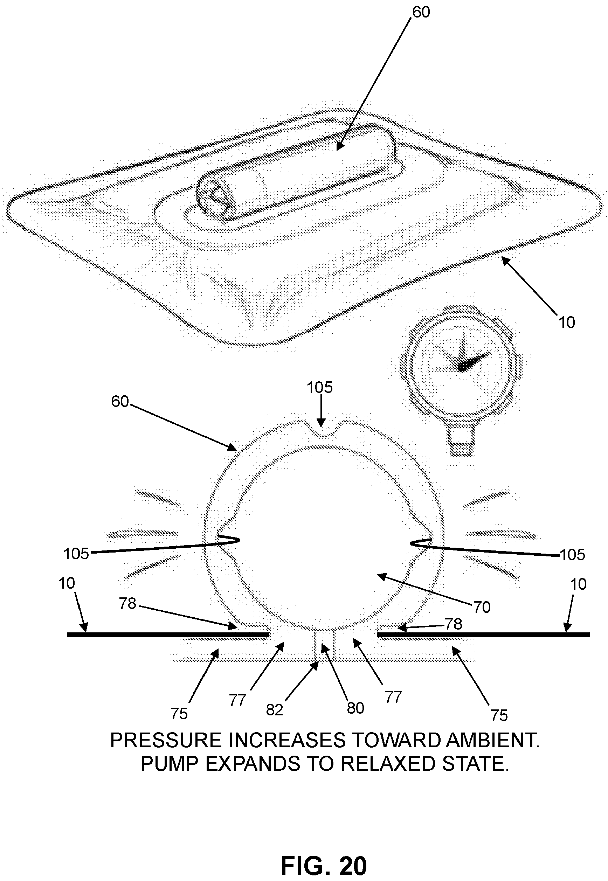

By way of example but not limitation, and looking now at Figs. 17-20, notches 105 can be formed in pump body 60 (e.g., at the "9 o'clock", "12 o'clock" and "3 o'clock" positions) so as to enhance the substantially "binary state" behavior of the pump body by further inducing pump body 60 to assume only its substantially fully expanded configuration or its substantially fully collapsed configuration. Note that the more that pump body 60 exhibits true "binary state" behavior, the more that pump efficiency will improve and the better that pump assembly 15 will serve as an automatic pressure indicator and as an automatic pressure limiter.

### NPWT Bandage Incorporating Gauze (Or Other Absorbent Wound Dressing) And Utilizing An Improved Pump Assembly

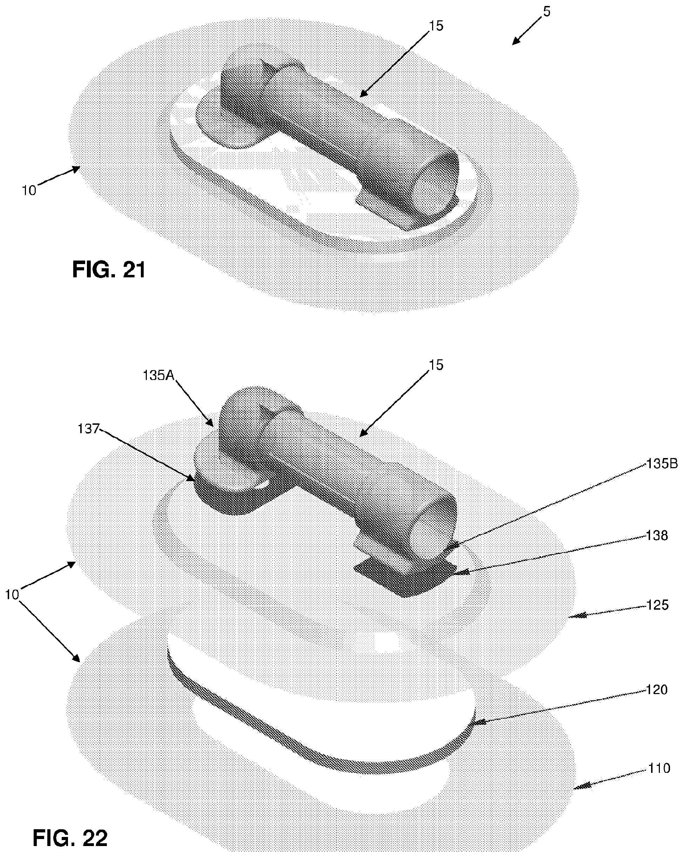

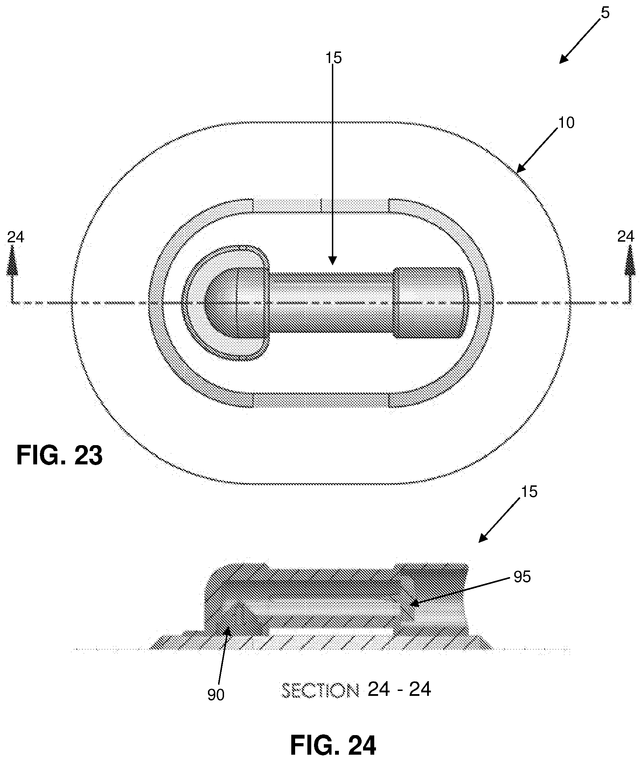

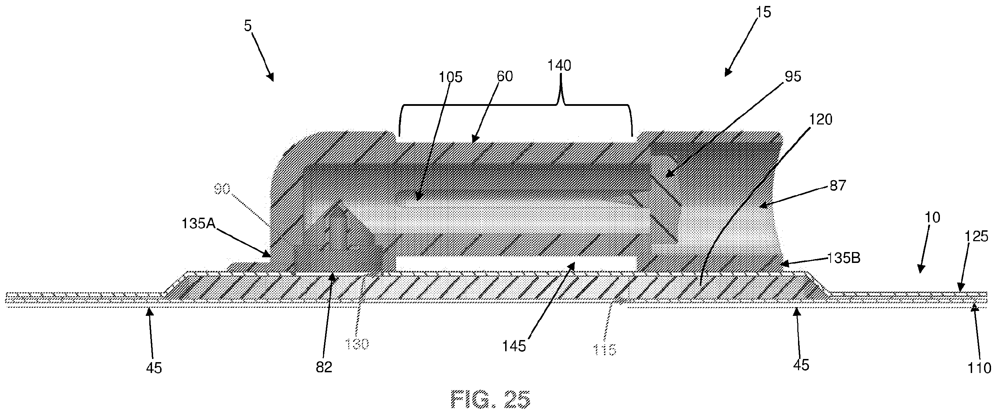

Looking next at Figs. 21-25, there is shown the negative pressure wound therapy (NPWT) bandage 5 formed in accordance with the present invention.

The NPWT bandage 5 shown in Figs. 21-25 is substantially the same as the NPWT bandage 5 shown in Figs. 1-16, and the NPWT bandage 5 shown in Figs. 17- 20, except that (i) in the construction shown in Figs. 21-25, membrane 10 comprises multiple layers which incorporate gauze (or other absorbent wound dressing), and (ii) in the construction shown in Figs. 21-25, pump assembly 15 has a modified construction and is secured to membrane 10 using a different approach.

More particularly, in this form of the invention, membrane 10 comprises a lower skin-contacting polyurethane layer 110 having a center opening 115, an intermediate foam (or gauze or other absorbent wound dressing) layer 120 for disposition over center opening 115 of lower skin-contacting polyurethane layer 110, and an upper polyurethane layer 125 for disposition over intermediate foam layer 120 and lower skin-contacting polyurethane layer 110. In the preferred form of the invention, upper polyurethane layer 125 is formed out of a substantially air- impermeable material. And, in the preferred form of the invention, upper polyurethane layer 125 and lower skin-contacting polyurethane layer 110 have the same size outer perimeter, so that upper polyurethane layer 125 does not contact the skin of the patient. The outer perimeters of upper polyurethane layer 125 and lower skin-contacting polyurethane layer 110 are secured to one another, capturing intermediate foam layer 120 therebetween. Intermediate foam layer 120 has an outer perimeter which is (i) larger than the perimeter of center opening 115 of lower skin contacting polyurethane layer 110, and (ii) smaller than the outer perimeter of the outer perimeters of lower skin-contacting polyurethane layer 110 and upper polyurethane layer 125. In this way, when NPWT bandage 5 has its center opening 115 of lower skin contacting polyurethane layer 110 positioned over a wound, fluid from the wound can pass through center opening 115 of lower skin-contacting polyurethane layer 110 to reach intermediate foam layer 120. It will be appreciated that adhesive 45 is positioned on the wound side surface of lower skin-contacting polyurethane layer 110 so that a substantially air tight seal may be established by NPWT bandage 5 about the perimeter of a wound (i.e., so as to form the aforementioned wound chamber) . An opening 130 is formed in upper polyurethane layer 125, and overlaps center opening 115 of lower skin-contacting polyurethane layer 110, so that wound-side passageway 80 of pump assembly 15 can access fluid (e.g., air, liquid, etc.) within the wound chamber (i.e., via opening 130 in upper polyurethane layer 125, the openings in intermediate foam layer 120, and center opening 115 of lower skin-contacting polyurethane layer 110) for evacuation during pumping of pump assembly 15.

The pump assembly 15 utilized in the NPWT bandage 5 of Figs. 21-25 is generally similar to the pump assembly 15 described above, except that it comprises a pair of pedestals 135A, 135B for mounting pump assembly 15 to membrane 10. More particularly, pedestal 135A comprises one end of pump body 60 and is adhered (e.g., by an adhesive 137) to the upper surface of membrane 10 (i.e., to the upper surface of upper polyurethane layer 125) so that wound-side passageway 80 and wound-side one-way valve 90 are aligned with opening 130 in upper polyurethane layer 125 (and hence in fluid communication with the wound chamber) . Pedestal 135B comprises the other end of pump body 60 and is adhered (e.g., by an adhesive 138) to the upper surface of membrane 10 (i.e., to the upper surface of upper polyurethane layer 125) . The intervening portion 140 of pump body 60 sits suspended between pedestal 135A and pedestal 135B, elevated above upper polyurethane layer 125 of membrane 10, so that a space 145 is formed between intervening portion 140 of pump body 60 and upper polyurethane layer 135 of membrane 10. Inasmuch as intervening portion 140 of pump body 60 is not mounted directly to membrane 10, but is instead suspended above membrane 10 by means of pedestals 135A and 135B, intervening portion 140 of pump body 60 can be formed with a true circular cross-section, whereby to enhance the substantially "binary state" behavior of the NPWT bandage. It will be appreciated that pump body 60 may incorporate one or more of the aforementio ned notches 105 so as to further enhance the substantially "binary state" behavior of the NPWT bandage.

Modifications Of The Preferred Embodiments It should be understood that many additional changes in the details, materials, steps and arrangements of parts, which have been herein described and illustrated in order to explain the nature of the present invention, may be made by those skilled in the art while still remaining within the scope of the invention as defined in the claims.

## Claims

1. A negative pressure wound therapy (NPWT) bandage for applying negative pressure to a wound, said NPWT bandage comprising:
a membrane configured for disposition over a wound so as to form a wound chamber between said membrane and the wound, said membrane comprising a wound-side surface, an atmosphere-side surface, and an opening extending through said membrane from said wound-side surface to said atmosphere-side surface; and
a pump assembly carried by said membrane, said pump assembly comprising:
a pump body comprising a resilient outer wall defining a single inner hollow chamber;
a wound-side passageway extending through said resilient outer wall and communicating with the wound chamber through said opening formed in said membrane;
a wound-side one-way valve disposed in said wound-side passageway, said wound-side one-way valve being configured to allow fluid to flow through said wound-side passageway from the wound chamber to said inner hollow chamber but to prevent fluid from flowing through said wound-side passageway from said inner hollow chamber to the wound chamber;
an atmosphere-side passageway extending through said resilient outer wall and connecting said inner hollow chamber and the atmosphere; and
an atmosphere-side one-way valve disposed in said atmosphere-side passageway, said atmosphere-side one-way valve being configured to allow fluid to flow through said atmosphere-side passageway from said inner hollow chamber to the atmosphere but to prevent fluid from flowing through said atmosphere-side passageway from the atmosphere to said inner hollow chamber,
wherein (i) when a compressive force is applied to said resilient outer wall of said pump body, fluid within said inner hollow chamber is forced out of said inner hollow chamber via said atmosphere-side passageway, and (ii) when the compressive force applied to said resilient outer wall of said pump body is thereafter reduced, fluid within the wound chamber is drawn into said inner hollow chamber through said wound-side passageway,
wherein said pump assembly is mounted to said membrane by a pair of pedestals, wherein one of the pair of pedestals comprises said wound-side passageway, and
wherein said pump body is suspended between said pair of pedestals and spaced from said membrane.

2. An NPWT bandage according to claim 1, wherein, when a pressure differential between a pressure within said inner hollow chamber and an atmospheric pressure is below a predetermined threshold, said pump body of said pump assembly assumes a substantially fully expanded configuration, and
wherein, when said pressure differential between the pressure within said inner hollow chamber and the atmospheric pressure is above said predetermined threshold, said pump body of said pump assembly assumes a substantially fully collapsed configuration.

3. An NPWT bandage according to claim 2 wherein said pump body abruptly changes state between said substantially fully expanded configuration and said substantially fully collapsed configuration, and between said substantially fully collapsed configuration and said substantially fully expanded configuration, as said pressure differential crosses said predetermined threshold so as to effectively constitute a substantially "binary state" device.

4. An NPWT bandage according to claim 2, wherein said predetermined threshold is between about 60 mm Hg and about 180 mm Hg.

5. An NPWT bandage according to claim 1 or 2, wherein said pump body comprises a substantially circular cross-section.

6. An NPWT bandage according to claim 2, wherein (i) said resilient outer wall of said pump body and said inner hollow chamber comprise a substantially circular cross-section when said pressure differential between the pressure within said inner hollow chamber and atmospheric pressure is below said predetermined threshold, and (ii) said resilient outer wall of said pump body bows inwardly when said pressure differential between the pressure within said inner hollow chamber and atmospheric pressure exceeds said predetermined threshold.

7. An NPWT bandage according to claim 1 or 2, wherein said wound-side surface of said membrane comprises an adhesive.

8. An NPWT bandage according to claim 7, wherein a release liner is disposed on said wound-side surface of said membrane atop said adhesive.

9. An NPWT bandage according to claim 1 or 2, wherein said pump assembly comprises a flange connected to said pump body.

10. An NPWT bandage according to claim 9, wherein said flange is formed integral with said pump body.

11. An NPWT bandage according to claim 1 or 2, wherein said inner hollow chamber is free of any solid matter.

12. An NPWT bandage according to claim 1 or 2, wherein said pump body comprises a substantially cylindrical configuration.

13. An NPWT bandage according to claim 1 or 2, wherein said membrane comprises a plurality of layers.

14. An NPWT bandage according to claim 13 wherein said membrane comprises a substantially air-impermeable layer and an absorbent layer, wherein said absorbent layer is disposed on the wound side of said substantially air-impermeable layer.

15. An NPWT bandage according to claim 13 further comprising an additional layer, wherein said absorbent layer is disposed between said substantially air-impermeable layer and said additional layer.

16. An NPWT bandage according to claim 10 wherein a neck is formed between said pump body and said flange, and further wherein a pair of recesses extend inwardly between said pump body and said flange.

## Patentansprüche

1. Unterdruck-Wundtherapieverband zur Anwendung von Unterdruck auf eine Wunde, wobei der Unterdruck-Wundtherapieverband umfasst: eine Membran, die dazu ausgebildet ist, über einer Wunde angeordnet zu werden, um zwischen der Membran und der Wunde eine Wundkammer zu bilden, wobei die Membran eine wundseitige Oberfläche, eine atmosphärenseitige Oberfläche und eine sich durch die Membran von der wundseitigen Oberfläche bis zur atmosphärenseitigen Oberfläche erstreckende Öffnung umfasst, und eine von der Membran getragene Pumpenanordnung, wobei die Pumpenanordnung umfasst: einen Pumpenkörper mit einer elastischen Außenwand, die eine einzige innere Hohlkammer begrenzt, einen sich durch die elastische Außenwand erstreckenden wundseitigen Durchgang, der über die in der Membran ausgebildete Öffnung mit der Wundkammer kommuniziert, ein in dem wundseitigen Durchgang angeordnetes wundseitiges Rückschlagventil, wobei das wundseitige Rückschlagventil dazu ausgebildet ist, Flüssigkeit durch den wundseitigen Durchgang von der Wundkammer zu der inneren Hohlkammer strömen zu lassen, jedoch zu verhindern, dass Flüssigkeit durch den wundseitigen Durchgang von der inneren Hohlkammer zu der Wundkammer strömt, einen sich durch die elastische Außenwand erstreckenden atmosphärenseitigen Durchgang, der die innere Hohlkammer mit der Atmosphäre verbindet, und ein in dem atmosphärenseitigen Durchgang angeordnetes atmosphärenseitiges Rückschlagventil, wobei das atmosphärenseitige Rückschlagventil dazu ausgebildet ist, Flüssigkeit durch den atmosphärenseitigen Durchgang von der inneren Hohlkammer zu der Atmosphäre strömen zu lassen, jedoch zu verhindern, dass Flüssigkeit durch den atmosphärenseitigen Durchgang von der Atmosphäre zu der inneren Hohlkammer strömt, wobei (i) bei Aufbringen einer Druckkraft auf die elastische Außenwand des Pumpenkörpers Flüssigkeit innerhalb der inneren Hohlkammer über den atmosphärenseitigen Durchgang aus der inneren Hohlkammer herausgedrückt wird und (ii) bei anschließender Verringerung der auf die elastische Außenwand des Pumpenkörpers aufgebrachten Druckkraft Flüssigkeit innerhalb der Wundkammer durch den wundseitigen Durchgang in die innere Hohlkammer eingesaugt wird, wobei die Pumpenanordnung mittels eines Paars von Sockeln an der Membran montiert ist, wobei einer der beiden Sockel den wundseitigen Durchgang umfasst, und wobei der Pumpenkörper zwischen dem Paar von Sockeln aufgehängt und von der Membran beabstandet ist.

2. Unterdruck-Wundtherapieverband nach Anspruch 1, wobei der Pumpenkörper der Pumpenanordnung eine im Wesentlichen vollständig expandierte Konfiguration einnimmt, wenn eine Druckdifferenz zwischen einem Druck innerhalb der inneren Hohlkammer und einem Atmosphärendruck unterhalb eines vorbestimmten Schwellenwerts liegt, und wobei der Pumpenkörper der Pumpenanordnung eine im Wesentlichen vollständig kollabierte Konfiguration einnimmt, wenn die Druckdifferenz zwischen dem Druck innerhalb der inneren Hohlkammer und dem Atmosphärendruck oberhalb des vorbestimmten Schwellenwerts liegt.

3. Unterdruck-Wundtherapieverband nach Anspruch 2, wobei der Pumpenkörper bei Überschreiten des vorbestimmten Schwellenwerts durch die Druckdifferenz abrupt zwischen der im Wesentlichen vollständig expandierten Konfiguration und der im Wesentlichen vollständig kollabierten Konfiguration sowie zwischen der im Wesentlichen vollständig kollabierten Konfiguration und der im Wesentlichen vollständig expandierten Konfiguration wechselt, sodass er effektiv eine Vorrichtung mit im Wesentlichen binärem Zustand bildet.

4. Unterdruck-Wundtherapieverband nach Anspruch 2, wobei der vorbestimmte Schwellenwert zwischen etwa 60 mm Hg und etwa 180 mm Hg liegt.

5. Unterdruck-Wundtherapieverband nach Anspruch 1 oder 2, wobei der Pumpenkörper einen im Wesentlichen kreisförmigen Querschnitt aufweist.

6. Unterdruck-Wundtherapieverband nach Anspruch 2, wobei (i) die elastische Außenwand des Pumpenkörpers und die innere Hohlkammer einen im Wesentlichen kreisförmigen Querschnitt aufweisen, wenn die Druckdifferenz zwischen dem Druck innerhalb der inneren Hohlkammer und dem Atmosphärendruck unterhalb des vorbestimmten Schwellenwerts liegt, und (ii) sich die elastische Außenwand des Pumpenkörpers nach innen wölbt, wenn die Druckdifferenz zwischen dem Druck innerhalb der inneren Hohlkammer und dem Atmosphärendruck den vorbestimmten Schwellenwert überschreitet.

7. Unterdruck-Wundtherapieverband nach Anspruch 1 oder 2, wobei die wundseitige Oberfläche der Membran einen Klebstoff umfasst.

8. Unterdruck-Wundtherapieverband nach Anspruch 7, wobei auf der wundseitigen Oberfläche der Membran über dem Klebstoff eine Abziehfolie angeordnet ist.

9. Unterdruck-Wundtherapieverband nach Anspruch 1 oder 2, wobei die Pumpenanordnung einen mit dem Pumpenkörper verbundenen Flansch umfasst.

10. Unterdruck-Wundtherapieverband nach Anspruch 9, wobei der Flansch einstückig mit dem Pumpenkörper ausgebildet ist.

11. Unterdruck-Wundtherapieverband nach Anspruch 1 oder 2, wobei die innere Hohlkammer frei von festen Stoffen ist.

12. Unterdruck-Wundtherapieverband nach Anspruch 1 oder 2, wobei der Pumpenkörper eine im Wesentlichen zylindrische Konfiguration aufweist.

13. Unterdruck-Wundtherapieverband nach Anspruch 1 oder 2, wobei die Membran mehrere Schichten umfasst.

14. Unterdruck-Wundtherapieverband nach Anspruch 13, wobei die Membran eine im Wesentlichen luftundurchlässige Schicht und eine absorbierende Schicht umfasst, wobei die absorbierende Schicht auf der Wundseite der im Wesentlichen luftundurchlässigen Schicht angeordnet ist.

15. Unterdruck-Wundtherapieverband nach Anspruch 13, ferner umfassend eine zusätzliche Schicht, wobei die absorbierende Schicht zwischen der im Wesentlichen luftundurchlässigen Schicht und der zusätzlichen Schicht angeordnet ist.

16. Unterdruck-Wundtherapieverband nach Anspruch 10, wobei zwischen dem Pumpenkörper und dem Flansch ein Hals ausgebildet ist und wobei sich ferner ein Paar von Ausnehmungen zwischen dem Pumpenkörper und dem Flansch nach innen erstreckt.

## Revendications

1. Pansement de thérapie des plaies par pression négative destiné à appliquer une pression négative à une plaie, ledit pansement de thérapie des plaies par pression négative comprenant :
une membrane configurée pour être disposée sur une plaie de manière à former une chambre de plaie entre ladite membrane et la plaie, ladite membrane comprenant une surface côté plaie, une surface côté atmosphère et une ouverture s'étendant à travers ladite membrane depuis ladite surface côté plaie jusqu'à ladite surface côté atmosphère, et un ensemble pompe porté par ladite membrane, ledit ensemble pompe comprenant :
un corps de pompe comprenant une paroi extérieure résiliente définissant une unique chambre creuse interne,
un passage côté plaie s'étendant à travers ladite paroi extérieure résiliente et communiquant avec la chambre de plaie par l'intermédiaire de ladite ouverture formée dans ladite membrane,
une valve unidirectionnelle côté plaie disposée dans ledit passage côté plaie, ladite valve unidirectionnelle côté plaie étant configurée pour permettre à un fluide de s'écouler à travers ledit passage côté plaie depuis la chambre de plaie jusqu'à ladite chambre creuse interne, mais pour empêcher un fluide de s'écouler à travers ledit passage côté plaie depuis ladite chambre creuse interne jusqu'à la chambre de plaie, un passage côté atmosphère s'étendant à travers ladite paroi extérieure résiliente et reliant ladite chambre creuse interne à l'atmosphère, et une valve unidirectionnelle côté atmosphère disposée dans ledit passage côté atmosphère, ladite valve unidirectionnelle côté atmosphère étant configurée pour permettre à un fluide de s'écouler à travers ledit passage côté atmosphère depuis ladite chambre creuse interne vers l'atmosphère, mais pour empêcher un fluide de s'écouler à travers ledit passage côté atmosphère depuis l'atmosphère jusqu'à ladite chambre creuse interne, dans lequel (i) lorsqu'une force de compression est appliquée à ladite paroi extérieure résiliente dudit corps de pompe, le fluide contenu dans ladite chambre creuse interne est expulsé de ladite chambre creuse interne par l'intermédiaire dudit passage côté atmosphère, et (ii) lorsque la force de compression appliquée à ladite paroi extérieure résiliente dudit corps de pompe est ensuite réduite, le fluide contenu dans la chambre de plaie est aspiré dans ladite chambre creuse interne à travers ledit passage côté plaie, dans lequel ledit ensemble pompe est monté sur ladite membrane au moyen d'une paire de socles, l'un de ladite paire de socles comprenant ledit passage côté plaie, et
dans lequel ledit corps de pompe est suspendu entre ladite paire de socles et espacé de ladite membrane.

2. Pansement de thérapie des plaies par pression négative selon la revendication 1, dans lequel, lorsqu'une différence de pression entre une pression à l'intérieur de ladite chambre creuse interne et une pression atmosphérique est inférieure à un seuil prédéterminé, ledit corps de pompe dudit ensemble pompe adopte une configuration sensiblement complètement expansée, et dans lequel, lorsque ladite différence de pression entre la pression à l'intérieur de ladite chambre creuse interne et la pression atmosphérique est supérieure audit seuil prédéterminé, ledit corps de pompe dudit ensemble pompe adopte une configuration sensiblement complètement effondrée.

3. Pansement de thérapie des plaies par pression négative selon la revendication 2, dans lequel ledit corps de pompe change brusquement d'état entre ladite configuration sensiblement complètement expansée et ladite configuration sensiblement complètement effondrée, et entre ladite configuration sensiblement complètement effondrée et ladite configuration sensiblement complètement expansée, lorsque ladite différence de pression franchit ledit seuil prédéterminé, de manière à constituer effectivement un dispositif à état sensiblement binaire.

4. Pansement de thérapie des plaies par pression négative selon la revendication 2, dans lequel ledit seuil prédéterminé est compris entre environ 60 mm Hg et environ 180 mm Hg.

5. Pansement de thérapie des plaies par pression négative selon la revendication 1 ou 2, dans lequel ledit corps de pompe comprend une section transversale sensiblement circulaire.

6. Pansement de thérapie des plaies par pression négative selon la revendication 2, dans lequel (i) ladite paroi extérieure résiliente dudit corps de pompe et ladite chambre creuse interne présentent une section transversale sensiblement circulaire lorsque ladite différence de pression entre la pression à l'intérieur de ladite chambre creuse interne et la pression atmosphérique est inférieure audit seuil prédéterminé, et (ii) ladite paroi extérieure résiliente dudit corps de pompe se bombe vers l'intérieur lorsque ladite différence de pression entre la pression à l'intérieur de ladite chambre creuse interne et la pression atmosphérique dépasse ledit seuil prédéterminé.

7. Pansement de thérapie des plaies par pression négative selon la revendication 1 ou 2, dans lequel ladite surface côté plaie de ladite membrane comprend un adhésif.

8. Pansement de thérapie des plaies par pression négative selon la revendication 7, dans lequel un film de protection amovible est disposé sur ladite surface côté plaie de ladite membrane, au-dessus dudit adhésif.

9. Pansement de thérapie des plaies par pression négative selon la revendication 1 ou 2, dans lequel ledit ensemble pompe comprend une bride reliée audit corps de pompe.

10. Pansement de thérapie des plaies par pression négative selon la revendication 9, dans lequel ladite bride est formée d'une seule pièce avec ledit corps de pompe.

11. Pansement de thérapie des plaies par pression négative selon la revendication 1 ou 2, dans lequel ladite chambre creuse interne est exempte de toute matière solide.

12. Pansement de thérapie des plaies par pression négative selon la revendication 1 ou 2, dans lequel ledit corps de pompe présente une configuration sensiblement cylindrique.

13. Pansement de thérapie des plaies par pression négative selon la revendication 1 ou 2, dans lequel ladite membrane comprend une pluralité de couches.

14. Pansement de thérapie des plaies par pression négative selon la revendication 13, dans lequel ladite membrane comprend une couche sensiblement imperméable à l'air et une couche absorbante, ladite couche absorbante étant disposée du côté plaie de ladite couche sensiblement imperméable à l'air.

15. Pansement de thérapie des plaies par pression négative selon la revendication 13, comprenant en outre une couche supplémentaire, ladite couche absorbante étant disposée entre ladite couche sensiblement imperméable à l'air et ladite couche supplémentaire.

16. Pansement de thérapie des plaies par pression négative selon la revendication 10, dans lequel un col est formé entre ledit corps de pompe et ladite bride, et dans lequel une paire d'évidements s'étend vers l'intérieur entre ledit corps de pompe et ladite bride.
